# EUROPÄISCHE PATENTSCHRIFT

(11) **EP 0 267 202 B1**
(45) Veröffentlichungstag und Bekanntmachung des Hinweises auf die Patenterteilung: **03.11.1993**
(21) Anmeldenummer: 87902307.5
(22) Anmeldetag: 21.04.1987
(51) Int. Cl.: A61B 17/11

(54) **VORRICHTUNG ZUR HERSTELLUNG EINER ANASTOMOSE**
ANASTOMOSIS DEVICE
DISPOSITIF D'ANASTOMOSE

(30) Priorität: 21.04.1986 AT 1052/86; 19.03.1987 AT 663/87
(43) Veröffentlichungstag der Anmeldung: 18.05.1988
(73) Patentinhaber: GLOBE CONTROL FINANZ AKTIENGESELLSCHAFT, FL-9490 Vaduz (LI)
(72) Erfinder: REDTENBACHER, Michael, A-1130 Wien (AT); MÜNCHOW, Karl, Heinz, A-9210 Pörtschach (AT)
(74) Vertreter: Gibler, Ferdinand, Dipl.Ing. Dr. techn.
(86) Internationale Anmeldenummer: AT8700029
(87) Internationale Veröffentlichungsnummer: WO8706448

(56) Entgegenhaltungen:
- NL-A- 7 711 347
- US-A- 3 193 165
- US-A- 4 351 466
- US-A- 4 576 167

## Beschreibung

Die Erfindung betrifft ein Klammernahtinstrument zur Durchführung einer Anastomose mit einem koaxial in einem Gehäuse angeordneten zentralen Rundstab, einem ein cirkuläres Skalpell aufweisenden, am distalen Gehäuseende befestigten Klammermagazin und einem Gegendruckkörper mit einer kalottenförmigen Außenfläche, einem Zapfen und einer senkrecht zum Zapfen verlaufenden, dem Klammermagazin zugewandten Klammerformungsebene, wobei der Zapfen mit dem zentralen Rundstab durch eine Steck- bzw. Gewindeverbindung lösbar festlegbar ist.

Ein Klammernahtinstrument dieser Art ist aus der NL-A-7711347 bekannt. Die US-A-4 351 466 offenbart ein Klammernahtinstrument mit einem Gegendruckkörper ohne Zapfen, der nicht zur separaten Einführung in ein zu anastomisierendes Organ ausgebildet ist.

Die Aufgabe der vorliegenden Erfindung liegt in der Schaffung eines Klammernahtinstrumentes, bei dem eine relativ einfache und problemlose Einführung des Klammermagazins und der Gegendruckplatte in das entsprechende Ende der zu anastomisierenden Organe durchführbar ist.

Diese Aufgabe wird erfindungsgemäß dadurch gelöst, daß das distale Ende des zentralen Rundstabes, an das der Zapfen mittels der Steck- bzw. Gewindeverbindung lösbar festlegbar ist, aus dem Klammermagazin vorsteht.

In weiterer Ausgestaltung der Erfindung kann vorgesehen sein, daß eine Abdeckkappe vorgesehen ist, die in Bezug auf die Klammerformungsebene nach außen gewölbt ist, und die die Klammerformungsebene und einen ersten im Gegendruckkörper befindlichen Teil der Steckverbindung abdeckt und über einen zweiten an der Abdeckkappe befindlichen Teil der Steckverbindung mit dem Gegendruckkörper verbindbar ist.

Durch die Vorordnung einer Abdeckkappe ist die Gegendruckplatte problemlos durch das zu anastomisierende Hohlorgan schiebbar. Durch die Steck- bzw. Gewindeverbindung am distalen Rundstabende ist nach Erreichen der vorgesehenen Position der Gegendruckplatte am Hohlorganende eine einfache und rasch durchführbare Loslösung der Abdeckkappe und anschließend ebenso eine rasche und sichere Verbindung zwischen Klammermagazin und Gegendruckplatte möglich.

Gemäß einer weiteren vorteilhaften Variante der Erfindung ist vorgesehen, daß die Steckverbindung als Bajonettverschluß ausgebildet ist. Ein derartiger Verschluß ist als Steckverbindung besonders geeignet, da mit einer lediglich geringen Drehung eines der zu verbindenden Teile eine sichere Verbindung herstellbar ist.

Gemäß einer weiteren vorteilhaften Ausführungsform der Erfindung ist vorgesehen, daß die Steckverbindung als mit dem Gegendruckkörper verbundener Zapfen mit einem pilzförmigen Ende und einem senkrecht zur Zapfenlängsrichtung abstehenden Positionierstift und als mit dem Ende des Rundstabes verbundener, einen Federring und eine schlitzförmige Öffnung aufweisender Rohrstutzen ausgebildet ist. Eine derartige Steckverbindung ist insoferne besonders vorteilhaft, als lediglich ein geringfügiges Ineinanderverschieben von Zapfen und Rohrstutzen ohne Drehung erforderlich ist. Der Positionierstift gewährleistet dabei in zuverlässiger Weise die richtige Lage der Gegendruckplatte zum Klammermagazin.

Die Steckverbindung kann entsprechend einer anderen vorteilhaften Ausführungsform der Erfindung auch als zapfenförmiger,über ein Gewinde lösbar mit dem Gegendruckkörper verbundener erster Steckverbindungsteil und als über ein Gewinde lösbar mit dem Rundstabende verbundener zweiter Steckverbindungsteil ausgebildet sein. Diese Steckverbindung eignet sich in vorteilhafter Weise als Nachrüstung für bereits im Einsatz befindliche, bekannte Klammernahtinstrumente. Eine derartige Nachrüstung ist mit nur geringem Aufwand verbunden und besonders einfach durchführbar.

Entsprechend einer vorteilhaften Variante der Erfindung kann die Abdeckkappe als paraboloidähnlicher Rotationskörper mit einer in Gebrauchslage der Klammerformungsebene bzw.-fläche des Gegendruckkörpers benachbarten, parallel dazu verlaufenden Grundebene ausgebildet sein, wobei die Grundebene eine senkrecht dazu und zentral zur Abdeckkappe verlaufende Bohrung mit einem Teil einer Steckverbindung, z.B. einer Führung für den Zapfen eines Bajonettverschlusses, aufweist. Eine derartig ausgebildete, paraboloidähnliche Abdeckkappe eignet sich besonders für eine schonende Einführung des Gegendruckkörpers in das zu anastomisierende Hohlorgan, wobei durch die zentral verlaufende Bohrung der von dem Gegendruckkörper abstehende und für die spätere Verbindung mit dem Klammermagazin benötigte Zapfen aufgenommen wird.

Als Verbindungsstück zur Verbindung von Klammermagazin und Abdeckkappe kann entsprechend einer anderen bevorzugten Ausführungsform der Erfindung auch ein zylindrisches Zwischenstück mit einem jeweils endseitig angeordneten Zapfen eines Bajonettverschlusses vorgesehen sein. Mit einem derartigen Zwischenstück kann in vorteilhafter Weise auch das Klammermagazin für deren einfachere und schonendere Einführung in das zu anastomisierende Hohlorgan mit einer Abdeckkappe verbunden werden.

Außerdem ist es auch eine vorteilhafte Weiterbildung der Erfindung, wenn die lösbar am Rundstabende oder der Abdeckkappe fixierbaren Verbindungsteile der Steckverbindung an ihrer Außenfläche eine geriffelte Oberfläche aufweisen. Eine derartig aufgerauhte Oberfläche der Verbindungsteile ermöglicht deren sichere Verbindung mit dem Rundstabende bzw. der Abdeckkappe ohne Zuhilfenahme eines Werkzeuges.

Eine weitere vorteilhafte Ausführung der Erfindung besteht aus den Merkmalen des Anspruches 9. Durch die nunmehr mögliche getrennte Einführung und auch getrennte Rückführung von Klammermagazin einerseits und Gegendruckkörper andererseits ist die Anastomose unter Vermeidung von Überbeanspruchungen bereits geknüpfter Tabaksbeutelnähte wesentlich einfacher durchführbar. Da nunmehr der Gegendruckkörper bereits in dem für diese Platte vorgesehenen Hohlorgan eingeführt wird, entfällt das bisher erforderliche und relativ umständliche Überstülpen des Hohlorganendes über den Gegendruckkörper, um anschließend die Naht knüpfen zu können. Von besonderem Vorteil ist jedoch, daß durch die erfindungsgemäße Vorrichtung nach der durchgeführten Anastomose der Gegendruckkörper unter Schonung der soeben geschaffenen Verbindungsstelle und der Hohlorganes selbst von dieser weg zum anderen Ausgang des entsprechenden Hohlorganes führbar ist. Gleichzeitig ist durch die Verbindung des Führungsdornes mit einem Endoskop der besondere Vorteil gegeben, daß die Stelle der Anastomisierung (z.B. Ösophagus) vor der Durchführung der Anastomose von innen her kontrollierbar ist. Weiters kann nach der Entfernung der das Klammermagazin tragenden Vorrichtung die soeben durchgeführte Anastomose optisch kontrolliert werden, um dabei etwaige Komplikationen festzustellen und zu beheben bzw. auch ein Foto zur Dokumentation anzufertigen.

Mit einer weiteren Ausbildung des Führungsdornendes bzw. des Endbereiches des Endoskops gemäß Anspruch 10 ist eine rasche Verbindung beider Teile herstellbar, ohne daß die Funktion des Endoskops beeinträchtigt wird. Ebenso ist nach durchgeführter Anastomose mit Hilfe der Rückholvorrichtung eine rasche Entkupplung herstellbar, so daß der Führungsdorn mitsamt der Vorrichtung und dem Klammermagazin über das gegenüberliegende Hohlorgan entfernbar ist.

Eine nach Anspruch 11 ausgebildeter Gegendruckkörper ist ohne besonderen konstruktiven Aufwand auch an bereits im Einsatz befindliche Endoskope zu befestigen, ohne daß deren Funktion beeinträchtigt wird.

Die nach Anspruch 15 vorgesehenen Maßnahmen ermöglichen auf sehr einfach Weise eine ständige Sichtkontrolle des Klammernahtinstrumentes sowohl beim Einführen, wie auch beim Nähen.

Im folgenden wird die Erfindung an Hand von in dem Zeichnung in beispielhaft dargestellten Ausführungsbeispielen näher beschrieben:

Es zeigen:
- Fig. 1: eine Seitenansicht eines bekannten Klammernahtinstrumentes,
- Fig. 2 und 3: eine perspektivische Darstellung eines Klammermagazines, eines Gegendruckkörpers und einer Abdeckkappe,
- Fig. 4 und 5: ebenfalls eine perspektivische Darstellung eines Gegendruckkörpers und einer Abdeckkappe gemäß einem anderen Ausführungsbeispiel,
- Fig. 6: ebenfalls eine perspektivische Darstellung eines Gegendruckkörpers und einer Abdeckkappe (Nahtkopf) gemäß wieder einem anderen Ausführungsbeispiel,
- Fig. 7: ein weiteres Ausführungsbeispiel zur Verbindung einer Abdeckkappe mit dem Rundstabende Klammernahtinstrumentes,
- Fig. 8: einen Längsquerschnitt durch ein erfindungsgemäß ausgebildetes Klammernahtinstrument,
- Fig. 9: einen Querschnitt durch einen mit einem Endoskop verbundenen Gegendruckkörper,
- Fig. 10 und 11: eine vergrößerte Detaildarstellung des Führungsdornes
- und Fig. 12: eine abgeänderte Ausführungsform eines Führungsdornes in Kombination mit einem Gegendruckkörper.

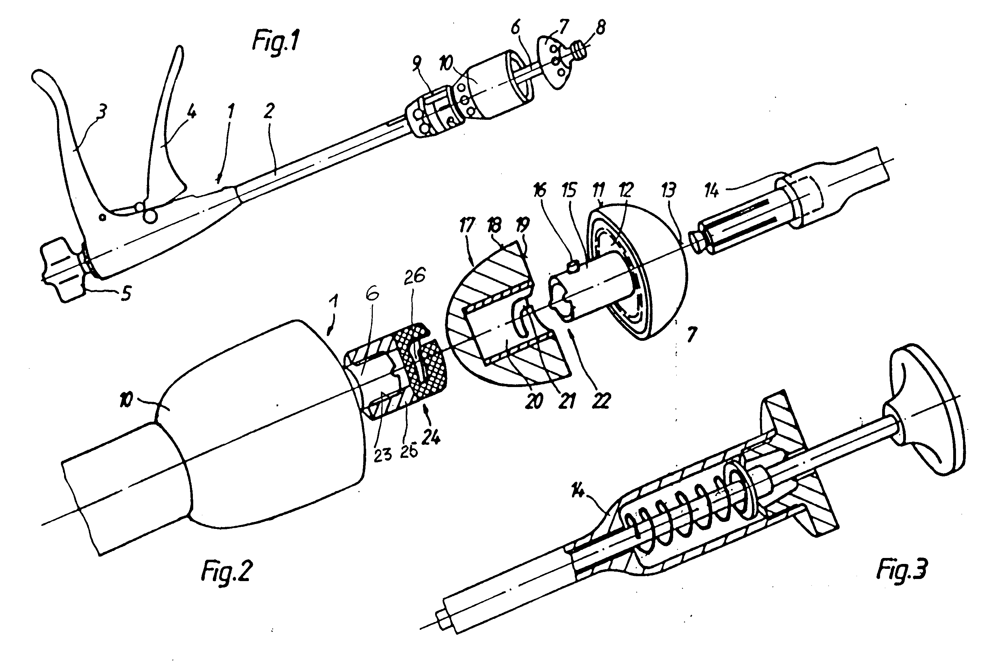

Ein in Fig. 1 ersichtliches Klammernahtinstrument 1 der bekannten Art setzt sich im wesentlichen aus einem zylindrischen Körper 2 und einem endseitig davon angeordneten Handgriff 3 mit einem vorschwenkbaren Hebel 4 und einer Flügelschraube 5 zusammen. Im zylindrischen Körper 2 ist ein koaxial geführter Rundstab 6 längsverschiebbar gelagert, an dessen Ende ein kalottenförmigen Gegendruckkörper 7 mit einer Schraubenmutter 8 lösbar befestigt ist. Am dem Handgriff 3 gegenüberliegenden Ende des zylindrischen Körpers 2 ist ein Drehverschluß 9 angeordnet, der mit einem zylindrischen Klammermagazin 10 lösbar verbunden ist. Zur Durchführung einer Anastomose müssen die zu anastomisierenden Hohlorganenden einerseits über das Klammermagazin 10 und andererseits über den Gegendruckkörper 7 geführt und im dazwischenliegenden Dornbereich mit Hilfe einer Tabaksbeutelnaht verknotet werden. Anschließend wird der Gegendruckkörper 7 durch Verdrehen der Flügelschraube 5 in Richtung zum Klammermagazin 10 bewegt. Mit der Betätigung des Hebels 4 werden im Klammermagazin 10 befindliche Klammern in die Hohlorganenden eingeführt und diese damit miteinander verbunden. Gleichzeitig werden die inneren Strukturen der Hohlorganenden mit den Tabaksbeutelnähten durch ein cirkuläres Skalpell abgetrennt.

Der in Fig.2 ersichtliche Gegendruckkörper 7 mit einer kalottenförmigen Außenfläche 11 und einer Klammerformungsebene 12 wird über die Bohrung 13 mit einer Sonde 14 und im Bereich der Klammerformungsebene 12 mit einem Zapfen 15 verbunden. Dieser weist an seinem freien Ende einen senkrecht zur Achsrichtung abstehenden Positionierstift 16 auf. Eine Abdeckkappe 17 mit einer paraboloidähnlichen, gewölbten Außenfläche 18 und einer Grundfläche 19 weist eine zentrale Bohrung 20 mit einer Bajonettführung 21 für den Positionierstift 16 auf. Führung 21 und Zapfen 15 bilden zusammen eine als Bajonettverschluß ausgebildete Steckverbindung 22.

Der aus dem Klammermagazin 10 vorstehende Rundstab 6 über ein Gewinde 23 mit einem als Steckverbindungsteil 24 ausgebildeten zylinderförmigen Teil 25 verbunden,der zur Aufnahme des Zapfens 15 vorgesehen ist und zur Aufnahme des Positionierstiftes 16 eine Bajonettführung 26 ausweist. Der Zapfen 15 der Abdeckkappe 7 bildet daher euch mit dem Rundstabende und dem Steckverbindungsteil 24 eine Steckverbindung 22.Zur Durchführung der Anastomose wird die bereits mit der Sonde 14 verbundene Abdeckkappe 7 über die Steckverbindung 22 mit der Abdeckkappe 17 verbunden, wobei die Grundfläche 19 der Abdeckkappe 17 auf der Klammerformungsebene 12 zu liegen kommt. Dieser eiförmige Kombinationskörper 7 und 17 wird mit Hilfe der Sonde ( das zur Einführung dienende Ende der Sonde 14 ist in Fig.3 dargestellt) in das zu anastomisierende Hohlorgan in Richtung zum eine Tabaksbeutelnaht aufweisenden Ende eingeführt. Sobald die richtige Position für den Gegendruckkörper 7 erreicht ist, wird die Abdeckkappe 17, die nunmehr aus dem Hohlorganende hervorsteht, durch eine Drehung um die Längsachse der Steckverbindung 22 von dem Gegendruckkörper 7 gelöst und entfernt. Das Klammernahtinstrument 1 wurde inzwischen in das andere Hohlorgan bis zur richtigen Position des Klammermagazins 10 eingeführt. Nach der Loslösung der Abdeckkappe 17 wird durch Einführung des Zapfens 15 in den zylinderförmigen Teil 25 und anschließender kurzer Drehung des Instrumentes 1 eine Verbindung zwischen dem Dornende und dem Gegendruckkörper 7 hergestellt. Anschließend erfolgt in bereits beschriebener und bekannter Weise die Verknotung der Hohlorganenden um den Rundstab 6. Nach durchgeführter Verbindung kann der Gegendruckkörper 7 unter Loslösung der Sonde 14 mit dem Klammernahtinstrument 1 bzw. unter Loslösung der Steckverbindung 22 wiederum über die Sonde 14 in umgekehrter Richtung aus dem Hohlorgan geführt werden.

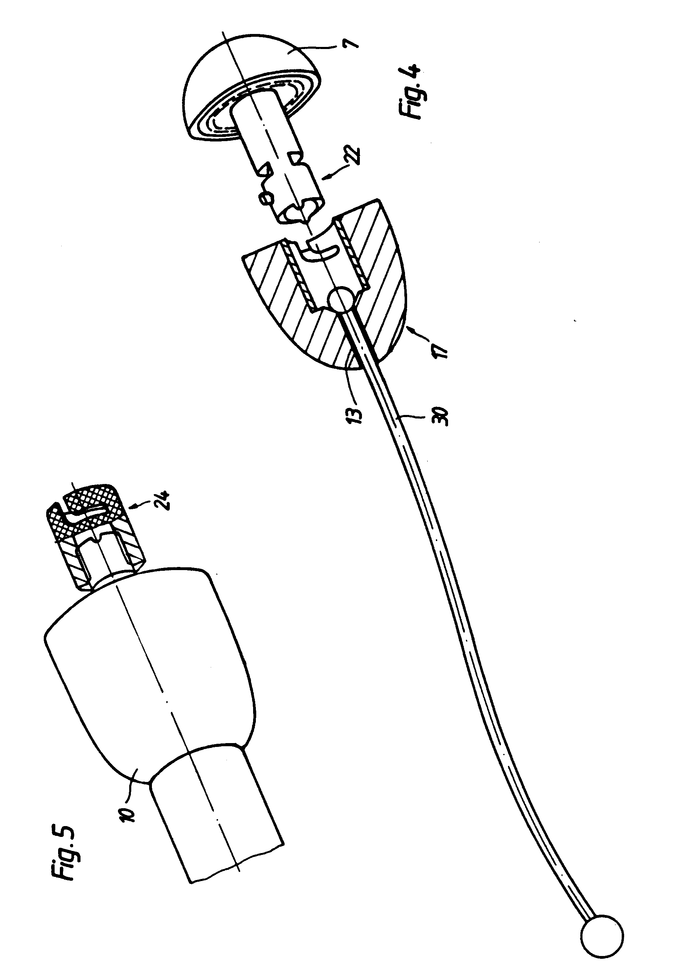

Der in Fig.4 dargestellte Gegendruckkörper 7 ist ebenfalls über eine als Bajonettverschluß ausgebildete Steckverbindung 22 mit der Abdeckkappe 17 verbindbar. Diese sind jedoch zusammen mit dem Gegendruckkörper 7 über ein in die Bohrung 13 eingeführtes Zugorgan 30 in Richtung zum zu anastomisierenden Hohlorganende gezogen. Nach Erreichen der vorgesehenden Position der Gegendruckkörpers 7 wird dieser wiederum über die Steckverbindung 22 mit dem in Fig.5 dargestellten Klammermagazin 10 verbunden.

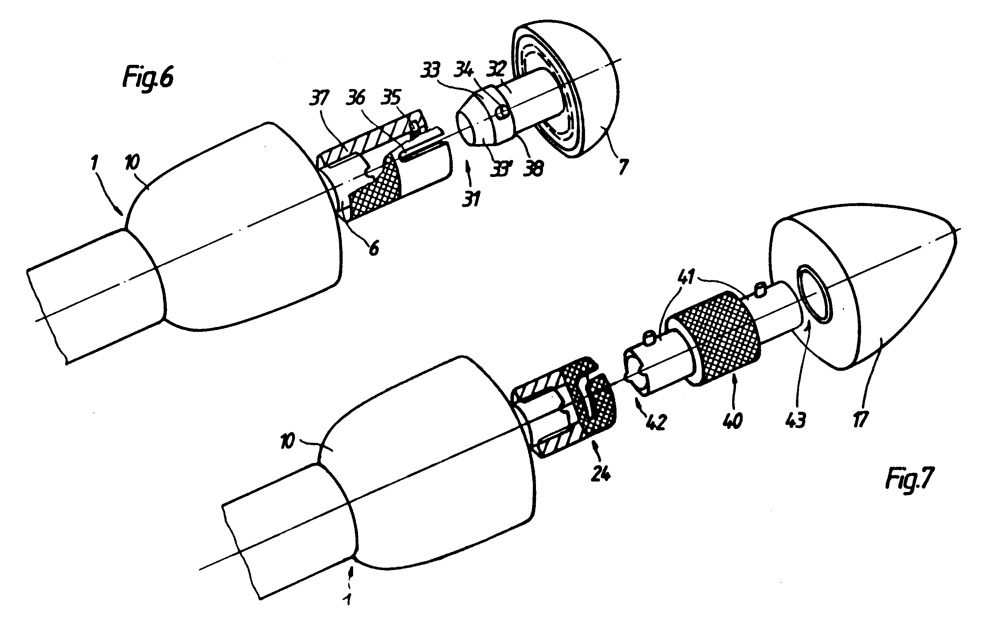

Eine in Fig.6 dargestellte Steckverbindung 31 ist als mit dem Gegendruckkörper 7 verbundener Zapfen 32 mit einem pilzförmigen Ende 33 und einem senkrecht zur Zapfenlängsrichtung abstehenden Positionierstift 34 und als mit dem Ende des Rundstabes 6 verbundener, einen Federring 35 und eine schlitzförmige Öffnung 36 aufweisender Rohrstutzen 37 ausgebildet.

Das pilzförmige Ende 33 weist einen kegelstumpfförmigen Ansatz 33' sowie eine Hinterschneidung 38 auf. Nach durchgeführter Verbindung beider Teile kommt der Federring 35 in der Hinterschneidung 38 zu liegen.

In Fig.7 ist ein zylindrisches Zwischenstück 40 ersichtlich, das jeweils endseitig einen Zapfan 41 aufweist. Jedes ist als Teil eines Bajonettverschlusses 42,43 einerseits mit der Abdeckkappe 17 und andererseits mit dem Ende des Rundstabes 6 verbindbar. Auf diese Weise kann auch das Rundstabende und das Klammermagazin 10 für eine schonende Einführung in das Hohlorgan abgedeckt werden.

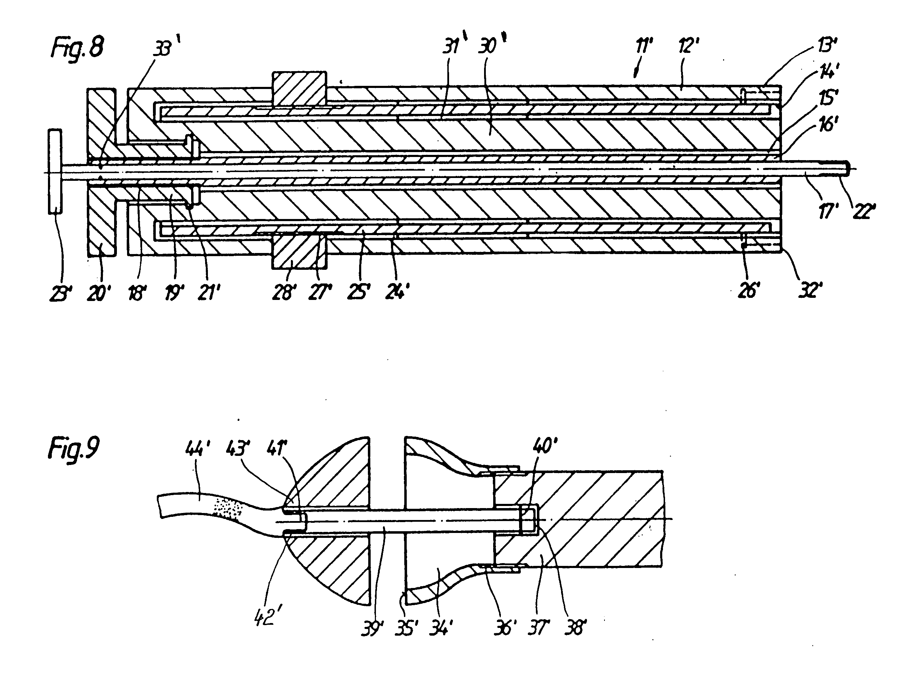

Der in Fig. 8 ersichtliche Teil des erfindungsgemäßen Klammernahtinstrumentes bzw. der Nahtmaschine 11' besteht aus einem zylindrischen Gehäuse 12', das endseitig mit einem Klammermagazin 13' und einem cirkulären Skalpell 14' verbunden ist. Zentral befindet sich in diesem zylindrischen Gehäuse 12' eine Bohrung 15'. In dieser Bohrung 15' wird längsverschieblich ein gegen Verdrehung gesicherter Hohldorn 16' geführt, in dessen Lumen sich ein Rundstab 17' befindet. Der Hohldorn 16' weist an einem Ende ein Gewinde 18' auf. Dieses greift in das Gewinde eines Zylinders 19', der an seinem Ende mit einer Flügelschraube 20' verbunden ist. Durch Betätigung dieser bei 21' drehbar im Gehäuse 12' gelagerten Flügelschraube 20' ist der Hohldorn 16' längsverschiebbar. Der in dem Hohldorn 16' geführte Rundstab 17' weist an dem Ende, an dem am Hohldorn die Flügelschraube 20' angebracht ist,eine Rändelschraube 23' und am gegenüberliegenden Ende ein Gewinde 22' auf. Statt dessen kann jedoch ebenso ein Steck- oder Bajonettverschluß vorgesehen sein. Der eben beschriebene Rundstab 17' dient der Verbindung mit einem in Fig. 9 noch näher zu beschreibenden Führungsdorn und einer Gegendruckplatte.

Zwischen der zentralen Bohrung 15' und der Zylindermantel-Außenfläche des Gehäuses 12' befindet sich eine zylinderförmige Öffnung 24', in der ein Führungszylinder 25' gelagert ist. Dieser steht über eine schematisch dargestellte Betätigungsvorrichtung 26' mit dem Klammermagazin 13' und dem Skalpell 14' in Verbindung. An der Mantelaußenseite des Führungszylinders 25' ist ein Gewinde 27' vorgesehen, das mit einem mit seiner Außenfläche außerhalb des Gehäuses 12' angeordneten Ringkörper 28' in Verbindung steht. Der Führungszylinder 25' weist in Achsrichtung verlaufende Schlitze auf, durch die die Gehäuseaußenwand mit dem Gehäuseinnenkörper 30' verbindende Stege 31' geführt sind. Diese Stege 31' dienen gleichzeitig auch dazu, eine Drehung des Führungszylinders 25' zu vermeiden. Durch Drehung des Ringkörpers 28' wird der Führungszylinder 25' verschoben. Dabei werden die Metallklammern 32' aus dem Klammermagazin 13' durch vorgesehene Schlitze und zugleich das Ringskalpell 14' zur Durchführung des Trennschnittes vorgeschoben. Der Rundstab 17' ist durch einen mit dem Zylinder 19' verbundenen Ring 33', der in eine entsprechende Ringnut des Rundstabes eingreift, zwar in Längsrichtung gesehen mit dem Zylinder 19' verbunden, jedoch gegenüber diesem durch die Rändelschraube 23' frei drehbar.

In Fig. 9 ist der zur Vorrichtung bzw. Nahtmaschine 11' gehörende Gegendruckkörper 34' ersichtlich. Dieser ist kalottenförmig ausgebildet und weist in dem der Anpreßfläche 35' für die Klammerformung gegenüberliegenden Endbereich eine ein Gewinde 36' aufweisende Öffnung zur lösbaren Fixierung des Endes eines Endoskops 37' auf. Die dargestellte Gewindeverbindung kann jedoch ebenso als Bajonettverschluß od. dgl. ausgebildet sein. Der Führungsdorn 39' weist endseitig einen Bajonettverschluß 40' auf, der in eine zentrale Bohrung 38' des Endoskops 37' einsteckbar ist. An seinem dem Verschluß 40' gegenüberliegenden Ende weist der Führungsdorn 39' ein Gewinde auf. In dieses Sackloch 41' ist aber auch ein Gewindezapfen einschraubbar. Dieser weist einen an die konvexe Oberfläche des Einführungskörpers 43' am zentral anschließenden Mandrin 44' und einen der Befestigungsstelle des Mandrins 44' gegenüberliegenden Gewindezapfen 42' zur Befestigung des Einführungskörpers 43' am Führungsdorn 39' auf.

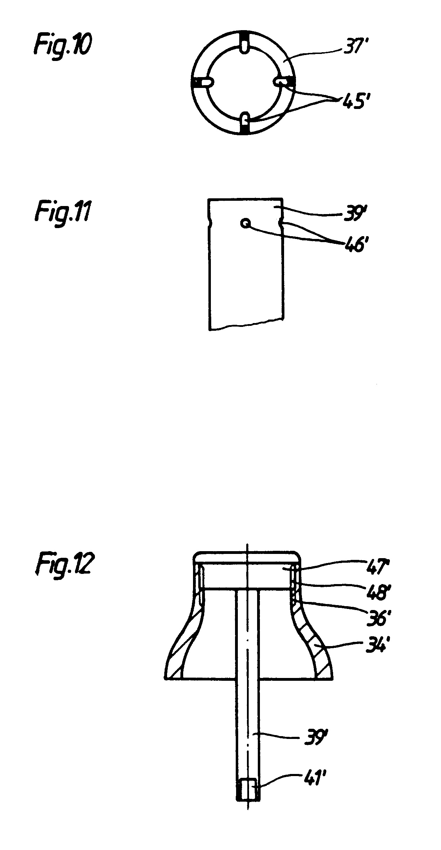

Wie in einer weiteren Ausführungsform gemäß Fig. 10 und 11 ersichtlich, sind im Endbereich eines Endoskops 37' vier Zapfen 45' derart angebracht, daß diese mit Federn in die Lichtung einer zentralen Bohrung des Endoskops 37' vorragen. Diese Zapfen 45' rasten in entsprechende, endseitig am Führungsdorn 39' vorgesehene Bohrungen 46' ein. Damit ist der Führungsdorn 39' rasch mit dem Endoskop 37' verbindbar. Soll der Führungsdorn 39' gelöst werden, wo werden die Zapfen 45' mit Hilfe einer Rückholvorrichtung aus der zentralen Bohrung des Endoskops 37' zurückgezogen.

In Fig. 12 ist ein etwas abgeänderter Führungsdorn 39' dargestellt, der dann eingesetzt wird, wenn die Nahtmaschine bzw. Vorrichtung 11' ohne Endoskop 37' angewendet werden soll. Der Führungsdorn 39' weist im dem Sackloch 41' gegenüberliegenden Endbereich einen Pfropfen 47' mit einem Gewinde 48' auf. Mit diesem ist der Pfropfen 47' in die ein Gewinde 36' aufweisende Öffnung des Gegendruckkörpers 34' einschraubbar.

Im folgenden wird die Wirkungsweise der erfindungsgemäß ausgebildeten Vorrichtung 1' anhand einer kreisrund genähten Anastomose mit dem Ösophagus näher beschrieben. Als erstes wird jeweils eine Tabaksbeutelnaht sowohl am Ösophagus als auch am zweiten Darmanteil genäht. Anschließend wird in die Bohrung 38' des Endoskops 37' der Führungsdorn 39' eingebracht. Dann wird die Gegendruckkörper 43' an das Endoskop 37' angeschraubt. Diese Vorbereitung wird auf einem sterilen Tisch durch einen Endoskopikar durchgeführt. Anschließend wird durch Einführen des Gewindezapfens 42' in das Sackloch 41' des Führungsdornes 39' der Einführungskörper 43' fixiert. Die 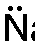 ahtmaschine 11' mit dem Klammermagazin 13' wird in den zweiten Darmanteil eingeführt, wobei um den Dorn 17' eine Tabaksbeutelnaht geknüpft wird. Anschließend wird das vorbereitete Endoskop 37' mit der Gegendruckplatte 34' mit Hilfe des Mandrins 44' durch den Mund eingeführt. Dabei wird der Mandrin 44' soweit vorgeschoben, bis der Operateur diesen am Ende des Ösophagus, an dem sich die Tabaksbeutelnaht befindet, zu fassen bekommt. Dabei wird mittels des Mandrin 44' der Gegendruckkörper 34' und das Endoskop 37' soweit vorgezogen, bis der Einführungskörper 43' über das Ende des Ösophagus hinaus vorgeschoben ist. Als nächstes erfolgt das Knüpfen der Tabaksbeutelnaht am Ösophagusende zwischen dem Einführungskörper 43' und dem Gegendruckkörper 34'. Sobald die beiden Tabaksbeutelnähte angefertigt sind, wird der Einführungskörper 43' vom Führungsdorn gelöst und entfernt. Anschließend wird der Rundstab 17' der Vorrichtung 11' mit dem Führungsdorn 39' verschraubt. Diese Verschraubung erfolgt durch Drehung des Rundstabes mit Hilfe der Rändelschraube 23'. Mit Hilfe des Endoskops 37' ist eine endoskopische Kontrolle der Ösophaguswand durchführbar. Durch Drehung des Ringkörpers 28' wird schließlich die Anastomisierung durchgeführt, wobei durch Vorschieben das Führungszylinders 25' die Klammern aus den entsprechenden Schlitzen geführt und die inneren Strukturen der Hohlorgane durch das zirkuläre Skalpell 14' abgetrennt werden. Durch eine Drehung der beiden verschraubten Teile 17' und 39' mit Hilfe der Rändelschraube 23' wird das Endoskop 37' abgekoppelt und die Nahtmaschine mitsamt dem Führungsdorn 39' aus dem Darm geführt. Zu diesem Zweck schließt der Bajonettverschluß 40' und das Gewinde 22' gegenläufig. Oberhalb der Anastomose verbleiben das Endoskop 37' und der an dieses angedockte Gegendruckkörper 34'. Mit dem Endoskop 37' wird eine Kontrolle der Anastomose durchgeführt. Dabei ist eine Behebung von etwaigen Komplikationen durch das Endoskop 37' durchführbar. Ebenso ist die Erstellung einer Fotodokumentation möglich.

Als weiteres Ausführungsbeispiel wird die Herstellung einer kreisrund genähten Anastomose mit dem Rektum beschrieben. Als erstes wird eine Tabaksbeutelnaht am Rektum und an dem mit dem Rektum zu vereinigenden Darmanteil durchgeführt. Anschließend wird der Führungsdorn 39' an den Gegendruckkörper 34' angedockt (Fig.12'). Der zu anastomisierende Darmanteil wird mit der Lichtung zum Operateur gehalten, und unter Aufsicht wird der Gegendruckkörper 34' in diese Lichtung eingeführt. Anschließend wird Unter Sicht die Tabakabeutelnaht geknüpft. Der das Klammermagazin tragende Teil der Nahtmaschine 11' wird am Dorn 13' mit dem aufblasbaren Einführungskörper 43' verbunden und durch den After eingeführt. Knüpfen der Tabaksbeutelnaht, Entfernung des Einführungskörpers 43'. Anschließend wird die Anastomisierung in der beschriebenen Art und Weise durchgeführt. Dabei ist von besonderem Vorteil, daß der Gegendruckkörper 34' unter Aufsicht in den Darm eingebracht und die Tabaksbeutelnaht unter Aufsicht geknüpft werden kann. Nachdem der Darm durch die Tabaksbeutelnaht verschlossen wurde, kann dieser problemlos in das kleine Becken geführt werden, ohne daß dabei Stuhl aus dem Darm ausrinnen kann.

## Patentansprüche

1. Klammernahtinstrument zur Durchführung einer Anastomose mit einem koaxial in einem Gehäuse (12') angeordneten zentralen Rundstab (6, 17'), einem ein cirkuläres Skalpell (14') aufweisenden, am distalen Gehäuseende befestigten Klammermagazin (13') und einem Gegendruckkörper (7, 34') mit einer kalottenförmigen Außenfläche (11), einem Zapfen (15, 32) und einer senkrecht zum Zapfen verlaufenden, dem Klammermagazin zugewandten Klammerformungsebene (12, 35'), wobei der Zapfen mit dem zentralen Rundstab (6, 17') durch eine Steck- bzw. Gewindeverbindung (22, 31, 22') lösbar festlegbar ist, dadurch gekennzeichnet, daß das distale Ende des zentralen Rundstabes (6, 17'), an das der Zapfen mittels der Steck- bzw. Gewindeverbindung lösbar festlegbar ist, aus dem Klammermagazin (13') vorsteht.

2. Klammernahtinstrument nach Anspruch 1, dadurch gekennzeichnet, daß eine Abdeckkappe (17, 43) vorgesehen ist, die in Bezug auf die Klammerformungsebene nach außen gewölbt ist, und die die Klammerformungsebene (12, 35') und einen ersten im Gegendruckkörper befindlichen Teil der Steckverbindung abdeckt und über einen zweiten an der Abdeckkappe befindlichen Teil der Steckverbindung mit dem Gegendruckkörper (7, 34') verbindbar ist.

3. Klammernahtinstrument nach Anspruch 1 oder 2, dadurch gekennzeichnet, daß die Steckverbindung (22) als Bajonettverschluß ausgebildet ist (Fig. 2).

4. Klammernahtinstrument nach Anspruch 1 oder 2, dadurch gekennzeichnet, daß die Steckverbindung (31) als mit dem Gegendruckkörper (7) verbundener Zapfen (32) mit einem pilzförmigen Ende (33) und einem senkrecht zur Zapfenlängsrichtung abstehenden Positionierstift (34) und als mit dem Ende des Rundstabes (6) verbundener, einen Federring (35) und eine schlitzförmige Öffnung (36) aufweisender Rohrstutzen (37) ausgebildet ist. (Fig. 6)

5. Klammernahtinstrument nach einem der Ansprüche 1, 2, 3 oder 4, dadurch gekennzeichnet, daß die Steckverbindung (22,31) als zapfenförmiger, über ein Gewinde lösbar mit dem Gegendruckkörper (7) verbundener erster Steckverbindungsteil (15) und als über ein Gewinde lösbar mit dem Rundstabende (6) verbundener zweiter Steckverbindungsteil (24) ausgebildet ist.

6. Klammernahtinstrument nach einem der Ansprüche 1 bis 5, dadurch gekennzeichnet, daß die Abdeckkappe (17,43') als paraboloidähnlicher Rotationskörper mit einer in Gebrauchslage der Klammerformungsebene bzw. -fläche des Gegendruckkörpers (7,34') benachbarten, parallel dazu verlaufenden Grundebene (19) ausgebildet ist, wobei die Grundebene (19) eine senkrecht dazu und zentral zur Abdeckkappe (17) verlaufende Bohrung (20) mit einen Teil einem Steckverbindung (22), z.B. einer Führung (21) für den Zapfen (15) eines Bajonettverschlusses aufweist.

7. Klammernahtinstrument nach einem der Ansprüche 1 bis 6, dadurch gekennzeichnet, daß als Verbindungsstück zur Verbindung von Klammermagazin und Abdeckkappe ein zylindrisches Zwischenstück (40) mit einen jeweils endseitig angeordneten Zapfen (41) eines Bajonettverschlusses vorgesehen ist (Fig. 7).

8. Klammernahtinstrument nach einem der Ansprüche 4 bis 6, dadurch gekennzeichnet, daß die lösbar am Rundstabende (6) oder der Abdeckkappe (17) fixierbaren Verbindungsteile (24,40) der Steckverbindung (22,31) an ihrer Außenfläche eine geriffelte Oberfläche aufweisen.

9. Klammernahtinstrument nach einem der Ansprüche 1 bis 8, dadurch gekennzeichnet, daß auf das aus dem Klammermagazin (13') ragende Ende des Rundstabes (17') ein Führungsdorn (39') koaxial aufschraubbar ist, der mit seinem anderen Ende an ein mit dem Gegendruckkörper (34') verbundenes Endoskop (37') ankuppelbar ist (Fig. 8 - 11).

10. Klammernahtinstrument nach Anspruch 9, dadurch gekennzeichnet, daß der Führungsdorn (39') in seinem an den Arbeitskanal (38') des Endoskops (37') anschließenden Endbereich senkrecht zur Stalemantelfläche verlaufende Bohrungen (46') aufweist, in die unter Vorspannung stehende, im Endbereich des Endoskops (37') angeordnete Zapfen (45') einrastbar sind, wobei die Zapfen (45') durch eine Rückholvorrichtung aus den Bohrungen (46') des Führungsdornes (39') rückholbar sind.

11. Klammernahtinstrument nach Anspruch 9 oder 10, dadurch gekennzeichnet, daß der Gegendruckkörper (34') in dem der Anpreßfläche (35') für die Klammerformung gegenüberliegenden Endbereich eine ein Gewinde (36'), einen Bajonettverschluß oder eine andere Schnellverbindung aufweisende Öffnung zur lösbaren Fixierung an Ende des Endoskops (37') aufweist.

12. Klammernahtinstrument nach Anspruch 9, 10 oder 11, dadurch gekennzeichnet, daß ein vorzugsweise aufblasbarer, kalottenförmiger Einführungskörper (43') mit einem an dessen konvexe Oberfläche zentral anschließenden Mandrin (44') und einem der Befestigungsstelle des Mandrins (44')gegenüberliegenden Gewindezapfen (41') zur Befestigung des Einführungskörpers (43') am Führungsdorn (39') vorgesehen ist (Fig. 9).

13. Klammernahtinstrument nach einem der Ansprüche 9 bis 12, dadurch gekennzeichnet, daß der in einem Hohldorn (16') gelagerte Rundstab (17') - im dem Klammermagazin (13') gegenüberliegenden Endbereich - eine senkrecht zur Längsachse verlaufende Scheibe (23') aufweist, und daß der Hohldorn (16') in diesem Endbereich über ein Gewinde (18') mit einer am Gehäuse (12') gelagerten Flügelschraube (20') in Verbindung steht (Fig. 8).

14. Klammernahtinstrument nach einem der Ansprüche 9 bis 13, dadurch gekennzeichnet, daß in einer zylindermantelförmigen Öffnung (24') des Gehäuses (12') ein endseitig mit dem cirkulären Skalpell (14') und über eine Betätigungsvorrichtung (26') mit dem Klammermagazin (13') in Verbindung stehender Führungszylinder (25') vorgesehen ist, der an seiner Mantelaußenseite ein Gewinde (27') aufweist, das mit einem mit seiner Außenfläche außerhalb des Gehäuses (12') angeordneten Ringkörper (28') im Eingriff steht (Fig. 8).

15. Klammernahtinstrument nach einem der Ansprüche 1 bis 14, dadurch gekennzeichnet, daß das Klammernahtinstrument mit einem Endoskop (37') verbunden ist.

## Claims

1. Clip suture instrument for carrying out an anastomosis, having a central round bar (6, 17') which is arranged coaxially in a housing (12'), a clip magazine (13') which has a circular scalpel (14') and is attached to the distal end of the housing, and a counter-pressure body (7, 34') with a dome-shaped outer surface (11), a pin (15, 32) and a clip shaping plane (12, 35') which runs perpendicular to the pin and faces the clip magazine, it being possible for the pin to be fixed releasably with the central round bar (6, 17') by a plug-in or threaded connection (22, 31, 22'), characterised in that the distal end of the central round bar (6, 17') can be fixed releasably to that of the pins by means of the plug-in or threaded connection from which the clip magazine (13') projects.

2. Clip suture instrument according to Claim 1, characterised in that a covering cap (17, 43) is provided which is curved outwards in relation to the clip shaping plane, and which covers the clip shaping plane (12, 35') and a first part of the plug-in connection located in the counter-pressure body and can be connected to the counter-pressure body (7, 34') via a second part of the plug-in connection located on the covering cap.

3. Clip suture instrument according to Claim 1 or 2, characterised in that the plug-in connection (22) is constructed as a bayonet closure (Figure 2).

4. Clip suture instrument according to Claim 1 or 2, characterised in that the plug-in connection (31) is constructed as a pin (32), which is connected to the counter-pressure body (7) and has a mushroom-shaped end (33) and a positioning pin (34) which projects perpendicular to the longitudinal direction of the pin, and as a tubular nozzle (37) which is connected to the end of the round bar (6) and has a spring ring (35) and a slot-shaped opening (36). (Figure 6).

5. Clip suture instrument according to one of Claims 1, 2, 3 or 4, characterised in that the plug-in connection (22, 31) is constructed as a pin-shaped, first plug-in connection part (15), which is connected releasably to the counter-pressure body (7) via a thread, and as a second plug-in connection part (24) which is connected releasably to the end of the round bar (6) via a thread.

6. Clip suture instrument according to one of Claims 1 to 5, characterised in that the covering cap (17, 43') is constructed as a paraboloid-type rotary body with a basic plane (19) which, in the position of use, is adjacent to the clip shaping plane or surface of the counter-pressure body (7, 34') and runs parallel thereto, the basic plane (19) having a bore (20), which runs perpendicular thereto and centrally relative to the covering cap (17) and has a part of a plug-in connection (22), for example a guide (21) for the pin (15) of a bayonet closure.

7. Clip suture instrument according to one of Claims 1 to 6, characterised in that a cylindrical transition piece (40) with a pin (41) of a bayonet closure is provided as connection piece for connecting the clip magazine and the covering cap, said pin being arranged at the end in each case (Figure 7).

8. Clip suture instrument according to one of Claims 4 to 6, characterised in that the connection parts (24, 40) of the plug-in connection (22, 31) which can be fixed releasably to the end of the round bar (6) or the covering cap (17) have a grooved surface on their outer surface.

9. Clip suture instrument according to one of Claims 1 to 8, characterised in that a guiding mandrel (39') can be screwed coaxially onto the end of the round bar (17') protruding from the clip magazine (13') and can be coupled by its other end to an endoscope (37') which is connected to the counter-pressure body (34') (Figures 8 - 11).

10. Clip suture instrument according to Claim 9, characterised in that the guiding mandrel (39') has bores (46') in its end region adjoining the operating channel (38') of the endoscope (37'), which bores run perpendicular to the outer surface of the bar and into which pins (45') can be engaged, said pins being prestressed, being arranged in the end region of the endoscope (37') and being retractable from the bores (46') of the guiding mandrel (39') by a retracting device.

11. Clip suture instrument according to Claim 9 or 10, characterised in that, in the end region located opposite the pressing-on surface (35') for the clip shaping, the counter-pressure body (34') has an opening, having a thread (36'), a bayonet closure or a different quick-acting connection, for the releasable fixture to the end of the endoscope (37').

12. Clip suture instrument according to Claim 9, 10 or 11, characterised in that a preferably inflatable, dome-shaped insertion body (43') with a mandrin (44') centrally adjoining its convex surface and a threaded pin (41') located opposite the attachment point of the mandrin (44') is provided for attachment of the insertion body (43') to the guiding mandrel (39') (Figure 9).

13. Clip suture instrument according to one of Claims 9 to 12, characterised in that the round bar (17') mounted in a hollow mandrel (16') - in the end region located opposite the clip magazine (13') - has a disc (23') running perpendicular to the longitudinal axis, and in that, in this end region, the hollow mandrel (16') is connected via a thread (18') to a wing screw (20') mounted on the housing (12') (Figure 8).

14. Clip suture instrument according to one of Claims 9 to 13, characterised in that a guide cylinder (25') is provided in a cylindrical opening (24') of the housing (12'), which guide cylinder is connected at the end to the circular scalpel (14') and via an actuating device (26') to the clip magazine (13') and has on its outer-surface side a thread (27') which is engaged with an annular body (28') arranged with its outer surface outside the housing (12') (Figure 8).

15. Clip suture instrument according to one of Claims 1 to 14, characterised in that the clip suture instrument is connected to an endoscope (37').

## Revendications

1. Appareil de suture par agrafe, pour effectuer une anastomose avec une barre cylindrique (6,17') centrale, disposée coaxialement dans un boitier (12'), un magasin à agrafes (13') présentant un scalpel (14') circulaire fixé sur l'extrémité distale du carter et un corps de contre-pression (7,34'), à surface extérieure (11) en forme de calotte, un tourillon (15,32) et un plan de formage d'agrafe (12,35') s'étendant perpendiculairement au tourillon, tourné vers le magasin à agrafes, le tourillon étant susceptible d'être fixé de façon amovible à la barre cylindrique centrale (6,17') au moyen d'une liaison à enfichage ou à vissage (22,31,32'), caractérisé en ce que l'extrémité distale de la barre cylindrique centrale (6,17'), sur laquelle le tourillon peut être fixé de façon amovible au moyen de la liaison à enfichage ou à vissage, fait saillie du magasin à agrafes (13').

2. Appareil de suture par agrafe selon la revendication 1, caractérisé en ce qu'est prévu un capuchon de recouvrement (17,43) incurvé vers l'extérieur par rapport au plan de formage des agrafes et qui recouvre le plan de formage des agrafes (12,35') et une première partie, se trouvant dans le corps de contre-pression, de la liaison à enfichage et qui peut être relié au corps de contre-pression (7,34') par l'intermédiaire d'une deuxième partie de la liaison d'enfichage se trouvant sur le capuchon de recouvrement.

3. Appareil de suture par agrafe selon la revendication 1 ou 2, caractérisé en ce que la liaison à enfichage (22) est réalisée sous forme de fermeture à baïonnette (figure 2).

4. Instrument de suture par agrafe selon la revendication 1 ou 2, caractérisé en ce que la liaison à enfichage (31) est réalisée sous forme d'un tourillon (32) relié au corps de contre-pression (7), avec une extrémité (33) en forme de champignon et une tige de positionnement (34) faisant saillie perpendiculairement par rapport à la direction longitudinale du tourillon et réalisée sous forme d'un embout tubulaire (37) relié à l'extrémité de la barre cylindrique (6), présentant une bague élastique (35) et une ouverture (36) en forme de fente (figure 6).

5. Appareil de suture par agrafe selon l'une des revendications 1, 2, 3, ou 4, caractérisé en ce que la liaison à enfichage (22,31) est réalisée sous forme d'une première partie de liaison à enfichage (15) en forme de tourillon reliée de façon amovible au corps de contre-pression (7), par l'intermédiaire d'un filetage et réalisée sous forme d'une deuxième partie de liaison à enfichage (24) reliée de façon amovible à la barre circulaire (6), par l'intermédiaire d'un filetage.

6. Appareil de suture par agrafe selon l'une des revendications 1 à 5, caractérisé en ce que le capuchon de recouvrement (17,43') est réalisé sous forme de corps de rotation paraboloïdal, avec un plan de base (19) qui, en position d'utilisation, est voisin du plan ou de la surface de formage des agrafes du corps de contre-pression (7,34') et s'étend parallèlement à celui-ci, le plan de base (19) présentant un perçage (20) s'étendant perpendiculairement par rapport à celui-ci et centralement par rapport au capuchon de recouvrement (17), avec une partie d'une liaison à enfichage (22), par exemple un guidage (21) pour le tourillon (15) d'une fermeture à baïonnette.

7. Appareil de suture par agrafe selon l'une des revendications 1 à 6, caractérisé en ce qu'est prévu comme pièce de liaison, pour assurer la liaison entre le magasin à agrafes et le capuchon de recouvrement, une pièce intermédiaire cylindrique (40) avec un tourillon disposé (41) à l'extrémité, pour une fermeture à baïonnette (figure 7).

8. Appareil de suture par agrafe, selon l'une des revendications 4 à 6, caractérisé en ce que les parties de liaison (24,40) pouvant être fixées de façon amovible sur l'extrémité de la barre cylindrique (6), ou le capuchon de recouvrement (17), de la liaison à enfichage (22,31) présentent sur leur face extérieure une surface cannelée.

9. Appareil de suture par agrafe, selon l'une des revendications 1 à 8, caractérisé en ce que, sur l'extrémité, sortant du magasin à agrafes (13'), de la barre cylindrique (17') est susceptible d'être vissée coaxialement un mandrin de guidage (39') pouvant être couplé par son autre extrémité à un endoscope (37') relié au corps de contre-pression (34') (figures 8 à 11).

10. Appareil de suture par agrafe, selon la revendication 9 caractérisé en ce que le mandrin de guidage (39') présente, dans sa zone d'extrémité raccordée au canal de travail (38') de l'endoscope (37'), des perçages (46') s'étendant perpendiculairement par rapport à la surface d'enveloppe de la barre, dans lesquelles peuvent être encliquetés des tourillons (45') placés sous précontrainte et disposés dans la zone d'extrémité de l'endoscope (37'), les tourillons (45') étant susceptibles d'être rappelés, au moyen d'un dispositif de rappel, hors des perçages (46') du mandrin de guidage (39').

11. Appareil de suture par agrafe, selon la revendication 9 ou 10, caractérisé en ce que le corps de contre-pression (34') présente, dans la zone d'extrémité en regard de la face de pressage (35') destinée au formage des agrafes, une ouverture qui présente un taraudage (36'), une fermeture à baïonnette ou une autre liaison rapide, en vue d'une fixation amovible à l'extrémité de l'endoscope (37').

12. Appareil de suture par agrafe, selon la revendication 9, 10 ou 11, caractérisé en ce qu'un corps d'introduction (43') en forme de calotte, de préférence pouvant être gonflé, avec un mandrin (44') raccordé centralement à sa surface convexe et un tourillon fileté (41') placé en face du point de fixation du mandrin (44') sont prévus pour assurer la fixation du corps d'introduction (43') sur le mandrin de guidage (39') (figure 9).

13. Appareil de suture par agrafe selon l'une des revendications 9 à 12, caractérisé en ce que la barre cylindrique (17') tourillonnant dans un mandrin creux (16') présente - dans la zone d'extrémité opposée au magasin à agrafes (13') - un disque (23') s'étendant perpendiculairement par rapport à l'axe longitudinal, et en ce que le mandrin creux (16') est relié, dans cette zone d'extrémité, à une vis à oreilles (20') tourillonnant sur le carter (12'), par l'intermédiaire d'un taraudage (18').

14. Appareil de suture par agrafe selon l'une des revendications 9 à 13, caractérisé en ce que, dans une ouverture (24'), en forme d'enveloppe cylindrique, du carter (12') est prévu un cylindre de guidage (25') relié à une extrémité au scalpel circulaire (14') et au magasin à agrafes (13'), par l'intermédiaire d'un dispositif d'actionnement (26'), ce cylindre de guidage (25') présentant, sur sa surface extérieure d'enveloppe, un filetage (27') venant en contact par sa surface extérieure avec un corps annulaire (28') disposé à l'extérieur du carter (12') (figure 8).

15. Appareil de suture par agrafe selon l'une des revendications 1 à 14, caractérisé en ce que l'appareil de suture à agrafe est relié à un endoscope (37').
